# EUROPÄISCHE PATENTANMELDUNG

(11) **EP 2 327 392 A2**
(43) Veröffentlichungstag der Anmeldung: **01.06.2011**
(21) Anmeldenummer: 10014975.6
(22) Anmeldetag: 25.11.2010
(51) Int. Cl.: A61K 8/19, A61K 8/42, A61K 8/44, A61K 8/49, A61K 8/64, A61K 8/67, A61K 8/97, A61Q 19/08, A61K 36/03, A61K 36/02, A61K 36/04, A61K 8/36

(54) **Kosmetische Wirkstoffzubereitung**

(30) Priorität: 27.11.2009 DE 102009055970
(71) Anmelder: Asam, Marcus, 85774 Unterföhring (DE); Asam, Mirjam, 85774 Unterföhring (DE); Ruth, Axel, 85774 Unterföhring (DE)
(72) Erfinder: Asam, Marcus, 85774 Unterföhring (DE)
(74) Vertreter: Witzany, Manfred

(57) **Zusammenfassung**

Eine kosmetische Wirkstoffzubereitung umfasst Extrakte bzw. deren Derivate, d. h. Meeresaster, Braunalgen, Korallenalgen und Knorpeltang. Zusätzlich sind in der kosmetischen Wirkstoffzubereitung noch metallgebundene Peptide enthalten. Diese Komponenten der kosmetischen Wirkstoffzubcrcitung verstärken sich gcgenseitig, so dass die gesamte Wirkstoffzubereitung günstige Feuchtigkeit spendende, Haut beruhigende und die Elastizität steigernde Wirkungen aufweist. Außerdem wird eine damit behandelte Haut vor schädlichen äußeren Umwelteinflüssen weitestgehend geschützt.

## Beschreibung

Die Erfindung betrifft eine kosmetische Wirkstoffzubereitung, welche insbesondere für Hautcreme eingesetzt wird.

Der Erfindung liegt die Aufgabe zugrunde, eine kosmetische Wirkstoffzubereitung zu schaffen, die sich durch eine Feuchtigkeit spendende, Haut beruhigende und die Elastizität steigernde Wirkung auszeichnet, wobei die Haut zusätzlich von schädlichen äußeren Umwelteinflüssen geschützt ist.

Diese Aufgabe wird erfindungsgemäß mit den Merkmalen des Patentanspruchs 1 gelöst.

Die kosmetische Wirkstoffzubereitung umfaßt Extrakte bzw. deren Derivate von Meerespflanzen sowie Meeresalgen.

Die Meeresaster (Tripleurospermum Maritima) wächst bevorzugt auf kargen, sandigen Hügeln an der bretonischen Atlantikküste. Diese Meerespflanze ist reich an den Vitaminen B5, B6, B3 und B9 sowie Mineralstoffen. Außerdem enthält sie eine hohe Konzentration an essentiellen Aminosäuren. Diese Stoffe bieten der Haut einen starken Schutz gegenüber freien Radikalen und wirken somit antioxidativ. Sie haben außerdem die Eigenschaft, entzündliche Prozesse in der Haut zu reduzieren, wodurch Fleckenbildung auf der Haut verringert wird. Vorzugsweise wird der Extrakt aus der Meeresaster derart gewonnen, dass die oben genannten Stoffe in möglichst hoher Konzentration im Extrakt enthalten sind.

Die Braunalge Alaria Esculenta kommt vorzugsweise aus der Bretagne und ist reich an ungesättigten Fettsäuren, insbesondere Omega 3, Omega 6 und Omega 9 Fettsäuren. Außerdem enthält sie Pflanzensterole und Lipoamide, die sich durch ihre antioxidative Wirkung auszeichnen. In der Braunalge enthaltene Wirkstoffe wirken in der Haut anregend für die Synthese von Glykosaminoglycanen, Hyaluronsäure und Proteoglycan. Dies wirkt damit dem Abbau von Elastin und Collagen entgegen und verbessert auf diese Weise die Elastizität der Haut. Außerdem steigern diese die Aktivität von Sirtuinen, welche der Hautalterung vorbeugen. Vorzugsweise wird der Extrakt aus der Braunalge derart gewonnen, dass die oben genannten Stoffe in möglichst hoher Konzentration im Extrakt enthalten sind.

Die Korallenalge (Corralina Officinalis) ist insbesondere reich an wichtigen Spurenelementen und Mineralien. Diese sind für die Aktivierung von Enzymen in der Haut von entscheidender Bedeutung, so dass die von den o. g. Extrakten geförderte Enzymbildung auch zu einer vermehrten Enzymaktivität führt. Vorzugsweise wird der Extrakt aus der Korallenalge derart gewonnen, dass die oben genannten Stoffe in möglichst hoher Konzentration im Extrakt enthalten sind.

Ein Knorpeltang (Chondrus Chrispus (Carrageenan)) enthält insbesondere ein natürliches Biopolymer, welches als Filmbildner wirkt und sich schützend auf die obersten Hautschichten legt. Dies verbessert insbesondere die Sensorik der Haut. Die Haut fühlt sich dabei weich an und erscheint gleichmäßiger. Damit schützt diese Wirkstoffkomponente zusätzlich die Haut vor schädigenden Umwelteinflüssen und verbessert deren Fähigkeit, Feuchtigkeit zu speichern. Vorzugsweise wird der Extrakt aus dem Knorpcltang derart gewonnen, dass die oben genannten Stoffe in möglichst hoher Konzentration im Extrakt enthalten sind.

Schließlich enthält die kosmetische Wirkstoffzubereitung noch an Metallpartikel gebundene Peptide. Diese Peptide haben vorzugsweise eine zu TGF-β ähnliche Aminosäuresequenz. Während die vorgenannten Wirkstoffkomponenten allesamt natürlichem, biologischem Ursprungs sind, ist diese Komponente künstlich synthetisiert. Diese zusätzliche Wirkstoffkomponente hat einen signifikanten Effekt auf die extrazelluläre Matrix, also die Zellzwischenräume. Außerdem steigert dieser Wirkstoff die natürliche Collagensynthese erheblich. Dies reduziert die tiefe Faltenbildung und verbessert die Hautelastizität erheblich. Durch die Anbindung der Peptide an Metallpartikel können die Peptide mit besonders hoher Konzentration in die Haut eingebracht werden, um auch tief liegende Falten abzubauen. Hierdurch ergibt sich ein besonders hoher Anti-Aging-Effekt. Vorzugsweise besitzen die Metallpartikel eine mittlere Größe von höchstens 2 µm, wobei die Anwendung von Nanopartikeln bevorzugt wird. Dies erleichLerL das Eindringen der Partikel in die Haut. Die Anbindung erfolgt vorzugsweise koordinativ in Form von Clustern oder kovalent, vorzugsweise über Sulfid- oder Disulfidbrücken.

Es hat sich nun herausgestellt, dass die einzelnen, oben genannten KomponenLen in erheblicher Wechselwirkung miteinander stehen. So wird beispielsweise die vom Meeresasterextrakt und vom Knorpeltangextrakt hervorgerufene Radikalfangwirkung durch die an die Metallpartikel gebundenen Peptide potenziert. Damit können die kostenintensiven biologischen Extrakte in wesentlich geringerer Konzentration eingesetzt werden als ohne die Peptid-Verstärker. Außerdem wird die Wirkung der gebundenen Peptide durch den Knorpeltangextrakt und den Korallenalgenextrakt verstärkt.

Bemerkenswerterweise stellte sich heraus, dass die oben beschriebene kosmetische Wirkstoffzubereitung eine erheblichc muskelentspannende Wirkung besitzt. Diese geht weit über das hinaus, was vom Meeresasterextrakt erwartet werden kann. Alle übrigen Wirkstoffe sollten grundsätzlich keinerlei muskelentspannende Wirkung besitzen. Es wird hierbei vermutet, dass Metallpartikel nach Abgabe der daran gebundenen Peptide eine verstärkende Wirkung auf den Meeresasterextrakt haben. Die muskelentspannende Wirkung ist für die kosmetische Wirkstoffzubereitung sehr positiv, da hierdurch ebenfalls Falten reduziert werden.

Für die Metallpartikel haben sich insbesondere Platin bzw. eine Platinlegierung bewährt. Platin bzw. Platinlegierungen haben die Eigenschaft, den Abbau der Peptide in der Haut durch Enzyme zu verhindern. Außerdem schützen Platin- bzw. Platinlegierungspartikel auch Enzyme aus den biologischen Extrakten vor einem unkontrollierten Abbau.

Für die Konzentrationen der einzelnen Wirkstoffkomponenten haben sich folgende Werte bewährt:
Für den Meeresasterextrakt 1 % bis 50 %, vorzugsweise zwischen 5 % und 30 %.
Für den Braunalgenextrakt 10 % bis 90 %, vorzugsweise zwischen 20 % und 70 %.
Für den Korallenalgenextrakt 2 % bis 50 %, vorzugsweise zwischen 5 % bis 30 %.
Für den Knorpeltangextrakt 2 % bis 30 %, vorzugsweise zwischen 5 % bis 15 % und
für an Metallpartikel gebundene Peptide 2 % bis 60 %, vorzugsweise zwischen 4 % bis 30 %.

Alle Prozentangaben sind als Gewichtsprozent zu verstehen.

### Beispiele:

### 1. Gesichtscreme

Eine Gesichtscreme besteht aus 54,6 % Wasser, 6 % Feuchthaltefaktor, 6 % Fettphasenverdicker, 23,8 % Ölen, 0,5 % Verdicker und 3,65 % Wirkstoffzubereitung.

Die Wirkstoffzubereitung selbst setzt sich dabei aus folgenden Wirkstoffen zusammen:
27 % Braunalgenextrakt, 27 % Korallenalgenextrakt, 14 % Knorpeltangextrakt, 28 % Meeresasterextrakt und 4 % an Platinpartikeln gebundene Peptide.

Diese Gesichtscreme wurde einer dermatologischen Untersuchung unterzogen. Dabei wurden 30 Testpersonen im Alter zwischen 50 und 70 Jahren mit gesunder Haut im Testareal eingesetzt. Die Testpersonen wiesen keinerlei chronische Hautentzündungen auf. Es wurde auch darauf geachtet, dass die Testpersonen keine Medikamerite einnehmen, die die Hautreaktionen beinträchtigen können. Sonnenbäder und Solariumbesuch wurden während der Durchführung der Studie ausgeschlossen.

Die Testpersonen wendeten die oben beschriebene Gesichtscreme täglich in einem vorgegebenen Areal an, welches vor Beginn der Teststudie und nach einem Monat mittels optischer 3D-Messung auf die Faltentiefe untersucht wurde.

In der folgenden Tabelle ist in der ersten Spalte die Nummer der Testperson, in der zweiten Spalte die gemessene Faltentiefe vor Anwendung der Gesichtscreme und in der dritten Spalte die gemessene Faltentiefe nach einmonatiger Anwendung der Gesichtscreme dargestellt. Die vierte Spalte zeigt schließlich die rechnerisch ermittelte, prozentuale Faltentiefe gemäß der Formel: Faltentiefe nachher / Faltentiefe vorher minus 1.

| | µm | µm | % |
|---|---|---|---|
| 1 | 158,9 | 73,0 | -54,1% |
| 2 | 166,2 | 83,2 | -49,9% |
| 3 | 191,1 | 93,3 | -51,2% |
| 4 | 152,3 | 65,3 | -57,1% |
| 5 | 164,4 | 85,5 | -48,0% |
| 6 | 170,7 | 80,9 | -52,6% |
| 7 | 192,3 | 76,0 | -60,5% |
| 8 | 167,4 | 69,7 | -58,4% |
| 9 | 159,0 | 73,1 | -54,0% |
| 10 | 175,5 | 86,8 | -50,5% |
| 11 | 168,8 | 80,2 | -52,5% |
| 12 | 171,4 | 89,1 | -48,0% |
| 13 | 205,7 | 100,2 | -51,3% |
| 14 | 170,6 | 86,6 | -49,2% |
| 15 | 156,2 | 58,2 | -62,7% |
| 16 | 171,2 | 69,6 | -59,3% |
| 17 | 164,7 | 71,9 | -56,3% |
| 18 | 185,0 | 85,5 | -53,8% |
| 19 | 181,3 | 83,8 | -53,8% |
| 20 | 192,9 | 113,7 | -41,1 % |
| 21 | 162,9 | 68,7 | -57,8% |
| 22 | 173,0 | 80,8 | -53,3% |
| 23 | 154,7 | 59,8 | -61,3% |
| 24 | 167,9 | 82,4 | -50,9% |
| 25 | 170,6 | 74,6 | -56,3% |
| 26 | 156,4 | 56,7 | -63,7% |
| 27 | 191,3 | 100,8 | -47,3% |
| 28 | 179,4 | 99,4 | -44,6% |
| 29 | 154,6 | 63,4 | -59,0% |
| 30 | 153,3 | 55,0 | -64,1% |
| | | | -54,1% |

Die ermittelte, prozentuale Faltentiefe wurde über die gesamten Testpersonen gemittelt, um eine statistisch verwertbare Aussage treffen zu können. Es ergibt sich demnach eine mittlere Faltentiefenreduktion um 54,1 % der ursprünglichen Faltentiefe.

### 2. Testformulierung A

Diese Testformulierung A entspricht der Gesichtscreme gemäß Punkt 1, wobei allerdings die an Platinpartikeln gebundenen Peptide ersatzlos weggelassen wurden. Diese Refcrcnzcreme A enthält demnach nur die Algenextrakte als Wirkstoff.

Die Anwendung der Testformulierung A erfolgte gleichzeitig mit der Gesichtscreme gemäß Punkt 1 von den gleichen Testpersonen, allerdings in einem anderen Areal. Dieses andere Areal wurde ebenfalls vor und einen Monat nach der Anwendung in gleicher Weise wie bei der Gesichtscreme gemäß Punkt 1 vermessen. Die Ergebnisse dieser Messung sind in der folgenden Tabelle dargestellt.

| | µm | µm | % |
|---|---|---|---|
| 1 | 159,1 | 118,6 | -25,5% |
| 2 | 178,6 | 133,8 | -25,1% |
| 3 | 189,4 | 156,3 | -17,5% |
| 4 | 158,0 | 116,9 | -26,0% |
| 5 | 177,4 | 128,6 | -27,5% |
| 6 | 171,2 | 130,7 | -23,7% |
| 7 | 193,1 | 132,4 | -31,4% |
| 8 | 157,9 | 125,6 | -20,5% |
| 9 | 146,3 | 118,3 | -19,1% |
| 10 | 182,6 | 128,2 | -29,8% |
| 11 | 169,7 | 137,3 | -19,1% |
| 12 | 168,7 | 131,4 | -22,1% |
| 13 | 205,8 | 156,4 | -24,0% |
| 14 | 173,1 | 126,3 | -27,0% |
| 15 | 156,6 | 116,0 | -25,9% |
| 16 | 174,6 | 140,6 | -19,5% |
| 17 | 170,3 | 127,3 | -25,2% |
| 18 | 188,6 | 148,2 | -21,4% |
| 19 | 189,0 | 151,4 | -19,9% |
| 20 | 181,0 | 142,6 | -21,2% |
| 21 | 161,9 | 118,0 | -27,1% |
| 22 | 167,7 | 135,4 | -19,3% |
| 23 | 156,9 | 120,9 | -22,9% |
| 24 | 160,6 | 126,5 | -21,2% |
| 25 | 175,0 | 137,1 | -21,7% |
| 26 | 158,3 | 108,7 | -31,3% |
| 27 | 200,0 | 161,9 | -19,1% |
| 28 | 190,2 | 137,0 | -28,0% |
| 29 | 159,1 | 111,8 | -29,7% |
| 30 | 147,5 | 107,6 | -27,1% |
| | | | -24,0% |

Aus dieser Testreihe ergibt sich eine mittlere Faltentiefenreduktion von 24,0 %, die somit deutlich unter der Faltentiefenreduktion der Gesichtscreme gemäß Punkt 1 liegt.

### 3. Testformulierung B

Als weitere Testformulierung B wurde die gleiche Zusammensetzung wie bei der Gesichtscreme gemäß Punkt 1 genutzt, wobei hier als Wirkstoff ausschließlich die an Platinpartikeln gebundenen Peptide in gleicher Konzentration eingesetzt wurden. Auch die Testformulierung B wurde von den gleichen Testpersonen wie die Gesichtscreme gemäß Punkt 1 unter den gleichen Testbedingungen geprüft, wobei wiederum ein anderes Hautareal verwendet wurde. Die Ergebnisse dieser Studie sind in der folgenden Tabelle zusammengefasst.

| | µm | µm | % |
|---|---|---|---|
| 1 | 156,9 | 126,0 | -19,7% |
| 2 | 164,7 | 151,7 | -7,9% |
| 3 | 196,9 | 163,6 | -16,9% |
| 4 | 165,9 | 127,3 | -23,3% |
| 5 | 162,8 | 134,1 | -17,6% |
| 6 | 159,0 | 135,8 | -14,6% |
| 7 | 203,7 | 162,3 | -20,3% |
| 8 | 164,1 | 139,7 | -14,9% |
| 9 | 161,6 | 148,9 | -7,9% |
| 10 | 176,1 | 155,5 | -11,7% |
| 11 | 161,3 | 127,5 | -21,0% |
| 12 | 183,2 | 139,6 | -23,8% |
| 13 | 223,0 | 185,6 | -16,8% |
| 14 | 174,9 | 149,5 | -14,5% |
| 15 | 152,4 | 120,0 | -21,3% |
| 16 | 169,4 | 150,0 | -11,5% |
| 17 | 156,6 | 133,7 | -14,6% |
| 18 | 194,0 | 160,6 | -17,2% |
| 19 | 174,0 | 134,4 | -22,8% |
| 20 | 186,4 | 160,0 | -14,2% |
| 21 | 157,8 | 122,6 | -22,3% |
| 22 | 187,1 | 158,2 | -15,4% |
| 23 | 153,2 | 120,0 | -21,7% |
| 24 | 169,6 | 146,7 | -13,5% |
| 25 | 171,3 | 152,3 | -11,1% |
| 26 | 152,9 | 115,7 | -24,3% |
| 27 | 184,6 | 157,4 | -14,7% |
| 28 | 183,3 | 140,4 | -23,4% |
| 29 | 166,5 | 133,4 | -19,9% |
| 30 | 155,5 | 134,8 | -13,3% |
| | | | -17,1% |

Bei dieser Studie ergibt sich eine mittlere Faltentiefenreduktion von 17,1 %. Auch dieser Wert liegt beträchtlich unter dem der Gesichtscreme gemäß Punkt 1. Bemerkenswerterweise ist die Summe der Faltentiefenreduktionen der beiden Testformulierungen A und B mit 41,1 % deutlich unter der Faltentiefenreduktionswirkung der Gesichtscreme gemäß Punkt 1 (54,1%). Die ermittelte Faltentiefenreduktion liegt demnach etwa 32% über dem Erwartungswert. Damit ist bewiesen, dass die Algenextrakte mit den an Platinpartikeln gebundenen Peptiden eine synergetische Wirkung eingehen, durch die sich die Wirkung der Gesamtformulierung über eine reine Summenwirkung hinaus steigern lässt. Diese synergetische Wirkung ist gegenwärtig noch nicht verstanden und wurde auch nicht erwartet.

Die Gesichtscreme gemäß Punkt 1 besitzt demnach eine überraschend stark Falten reduzierende Wirkung, außerdem wurde eine extrem hohe antioxidative Wirkung nachgewiesen.

### 4. Gesichtsserum

Ein Gesichtsserum besteht aus 80,7 % Wasser, 7,85 % Feuchthaltefaktoren, 3,5 % Verdicker, 2,6 % Ölen, 0,3 % Emulgatoren und 1,7 % Wirkstoffzubereitung.

Die Wirkstoffzubereitung selbst besteht wiederum aus: 59 % Braunalgenextrakt, 6 % Korallenalgenextrakt, 12 % Knorpeltangextrakt, 6 % Meeresasterextrakt und 17 % an Platinpartikel gebundene Peptide.

Dieses Gesichtsserum zeichnet sich durch eine hohe Antifaltenwirkung und eine hohe antioxidative Wirkung aus.

### 5. Augencreme

Eine Augencreme besteht aus 64,3 % Wasser, 16 % Feuchthaltefaktoren, 0,75 % Emulgatoren, 1,95 % Fettphasenverdicker, 5 % Ölen und 1,7 % Wirkstoffzubereitung.

Die Wirkstoffzubereitung selbst besteht wiederum aus:
59 % Braunalgenextrakt, 6 % Korallenalgenextrakt, 6 % Knorpeltangextrakt, 6 % Meeresasterextrakt und 23 % an Platinpartikel gebundene Peptide.

Diese Augencreme besitzt eine unerwartet hohe die Elastizität der Haut verbessernde Wirkung sowie eine sehr hohe Antifaltenwirkung.

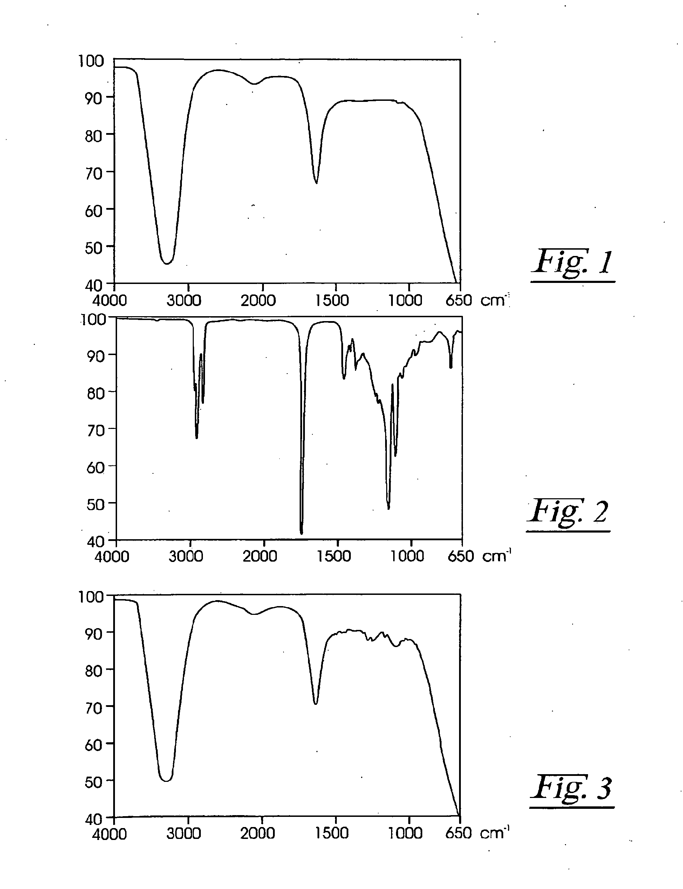

Die Figur 1 zeigt ein Infrarotspektrum des in den Beispielen eingesetzten Meeresasterextrakts.

Die Figur 2 zeigt ein Infrarotspektrum des in den Beispielen eingesetzten Braunalgenextrakts.

Die Figur 3 zeigt ein Infrarotspektrum des in den Beispielen eingesetzten Korallenalgenextrakts.

## Patentansprüche

1. Kosmetische Wirkstoffzubereitung, welche folgende Komponenten umfaßt:
a) mindestens einen Extrakt und/oder daraus hergestelltes Derivat aus einer Meeresaster,
b) mindestens einen Extrakt und/oder daraus hergestelltes Derivat aus einer Braunalge,
c) mindestens einen Extrakt und/oder daraus hergestelltes Derivat aus einer Korallenalge,
d) mindestens einen Extrakt und/oder daraus hergestelltes Derivat aus einem Knorpeltang und
e) an Metallpartikel gebundene Peptide.

2. Kosmetische Wirkstoffzubereitung nach Anspruch 1, **dadurch gekennzeichnet, dass** sie Vitamine B3, B5, B6 und/oder B9 aufweist.

3. Kosmetische Wirkstoffzubereitung nach Anspruch 1 oder 2, **dadurch gekennzeichnet, dass** sie essentielle Aminosäuren aufweist.

4. Kosmetische Wirkstoffzubereitung nach mindestens einem der Ansprüche 1 bis 3, **dadurch gekennzeichnet, dass** sie ungesättigte Fettsäuren, Omega 3, Omega 6 und/oder Omega 9 enthält.

5. Kosmetische Wirkstoffzubereitung nach mindestens einem der Ansprüche 1 bis 4, **dadurch gekennzeichnet, dass** sie Pflanzensterole, Lipoamide und/oder Spurenelemente enthält.

6. Kosmetische Wirkstoffzubereitung nach mindestens einem der Ansprüche 1 bis 5, **dadurch gekennzeichnet, dass** der Brauralgenextrakt aus der Alaria Esculenta gewonnen ist.

7. Kosmetische Wirkstoffzubereitung nach mindestens einem der Ansprüche 1 bis 6, **dadurch gekennzeichnet, dass** der Korallenalgenextrakt aus der Corralina Officinalis gewonnen ist.

8. Kosmetische Wirkstoffzubereitung nach mindestens einem der Ansprüche 1 bis 7, **dadurch gekennzeichnet, dass** die Metallpartikel Platin und/oder eine Platinlegierung aufweisen.

9. Kosmetische Wirkstoffzubereitung nach mindestens einem der Ansprüche 1 bis 8, **dadurch gekennzeichnet, dass** der Meeresasterextrakt und/oder dessen Derivat in der Wirkstoffzubereitung in einer Konzentration zwischen 1 % und 50 %, vorzugsweise zwischen 3 % und 30 % enthalten ist.

10. Kosmetische Wirkstoffzubereitung nach mindestens einem der Ansprüche 1 bis 9, **dadurch gekennzeichnet, dass** der Braunalgenextrakt und/oder dessen Derivat in der Wirkstoffzubereitung in einer Konzentration zwischen 10 % und 90 %, vorzugsweise zwischen 20 % und 70 % enthalten ist.

11. Kosmetische Wirkstoffzubereitung nach mindestens einem der Ansprüche 1 bis 10, **dadurch gekennzeichnet, dass** der Korallenalgenextrakt und/oder dessen Derivat in der Wirkstoffzubereitung in einer Konzentration zwischen 2 % und 50 %, vorzugsweise zwischen 5 % und 30 % enthalten ist.

12. Kosmetische Wirkstoffzubereitung nach mindestens einem der Ansprüche 1 bis 11, **dadurch gekennzeichnet, dass** der Knorpeltangextrakt und/oder dessen Derivat in der Wirkstoffzubereitung in einer Konzentration zwischen 2 % und 30 %, vorzugsweise zwischen 5 % und 15 % enthalten ist.

13. Kosmetische Wirkstoffzubereitung nach mindestens einem der Ansprüche 1 bis 12, **dadurch gekennzeichnet, dass** die an Metallpartikeln gebundenen Peptide in einer Konzentration zwischen 2 % und 60 %, vorzugsweise zwischen 4 % und 30 % enthalten ist.

14. Kosmetische Wirkskoffzubereitung nach mindestens einem der Ansprüche 1 bis 13, **dadurch gekennzeichnet, dass** mindestens einer der Extrakte und/oder das daraus hergestellte Derivat wenigstens teilweise aus einem Organismus gewonnen ist, der aus der Bretagne stammt.
